(19) Europäisches Patentamt
European Patent Office
Office européen des brevets

(11) **EP 3 233 081 B1**

(12) **EUROPEAN PATENT SPECIFICATION**

(45) Date of publication and mention
of the grant of the patent:
**22.09.2021   Bulletin 2021/38**

(51) Int Cl.:
*A61K 31/4468* (2006.01)    *A61K 9/70* (2006.01)
*A61K 47/14* (2017.01)    *A61P 25/04* (2006.01)
*A61P 23/00* (2006.01)

(21) Application number: **15871096.2**

(22) Date of filing: **17.12.2015**

(86) International application number:
**PCT/US2015/066453**

(87) International publication number:
**WO 2016/100708 (23.06.2016 Gazette 2016/25)**

(54) **TRANSDERMAL DRUG DELIVERY DEVICE INCLUDING FENTANYL**

TRANSDERMALE ARZNEIMITTELVERABREICHUNGSVORRICHTUNG ENTHALTEND FENTANYL

DISPOSITIF D'ADMINISTRATION TRANSDERMIQUE DE MÉDICAMENT, COMPRENANT DU FENTANYL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **19.12.2014   US 201462094659 P**

(43) Date of publication of application:
**25.10.2017   Bulletin 2017/43**

(73) Proprietor: **Kindeva Drug Delivery L.P.**
**St. Paul, MN 55170 (US)**

(72) Inventors:
• **PRESZLER PRINCE, Amy**
**Saint Paul, MN 55133-3427 (US)**
• **CANTOR, Adam, S.**
**Saint Paul, MN 55133-3427 (US)**
• **WOEHRLE, Stephen, J.**
**Saint Paul, MN 55133-3427 (US)**
• **HART, John, R.**
**Saint Paul, MN 55133-3427 (US)**
• **DIZIO, James, P.**
**Saint Paul, MN 55133-3427 (US)**
• **SYKORA, Sarah**
**Saint Paul, MN 55133-3427 (US)**
• **HUSBERG, Michael, L.**
**Saint Paul, MN 55133-3427 (US)**

(74) Representative: **Vossius & Partner**
**Patentanwälte Rechtsanwälte mbB**
**Siebertstrasse 3**
**81675 München (DE)**

(56) References cited:
**WO-A1-2009/052204        WO-A1-2009/052204**
**WO-A1-2011/010556        WO-A1-2012/012417**
**WO-A2-02/26217            WO-A2-03/097008**
**CN-B- 101 780 057         US-A- 4 588 580**
**US-A1- 2004 213 832       US-A1- 2011 020 407**
**US-A1- 2014 005 617       US-A1- 2014 243 765**
**US-B2- 8 778 382**

• **ROY S D ET AL: "CONTROLLED TRANSDERMAL DELIVERY OF FENTANYL: CHARACTERIZATIONS OF PRESSURE-SENSITIVE ADHESIVES FOR MATRIX PATCH DESIGN", JOURNAL OF PHARMACEUTICAL SCIENCES, AMERICAN CHEMICAL SOCIETY AND AMERICAN PHARMACEUTICAL ASSOCIATION, US, vol. 85, no. 5, 1 May 1996 (1996-05-01), pages 491-495, XP000583527, ISSN: 0022-3549, DOI: 10.1021/JS950415W**
• **None**

## Description

### Background

Transdermal drug delivery devices are designed to deliver a therapeutically effective amount of drug across the skin of a patient. Transdermal drug delivery devices typically involve a carrier (such as a liquid, gel, or solid matrix, or a pressure sensitive adhesive) into which the drug to be delivered is incorporated. Devices known to the art include reservoir type devices involving membranes that control the rate of drug release to the skin and devices where the drug is dispersed or dissolved in a matrix such as a pressure sensitive adhesive.

It has long been known that fentanyl is an extremely potent and effective anesthetic and analgesic. Fentanyl is most frequently administered as the citrate salt intravenously (IV) or intramuscularly (IM) to achieve therapeutic effects. Fentanyl citrate is preferred for injection because of its aqueous solubility. Fentanyl may also be administered as a transdermal patch or as a lozenge. Additional details regarding pharmacokinetics, uses, and dosages of fentanyl may be found in the monograph "Fentanyl Citrate", AHFS 98 Drug Information, ed.: G. K. McEvoy, American Society of Health-Systems Pharmacists, p.1677-1683 (1998).

Following IV or IM administration the onset of action is very rapid but the decrease in serum fentanyl concentration is also rapid, which necessitates dosing at frequent intervals. Minimum effective analgesic serum levels of fentanyl range from 0.2 to 2 ng/mL. Furthermore, oral absorption of fentanyl is low. Lozenges that provide a combination of transmucosal and oral dosage are indicated for treatment of breakthrough cancer pain, but also have a short duration of action.

Transdermal administration of fentanyl can overcome the drawbacks of frequent dosing needed with the aforementioned routes of administration. This can also avoid the peaks and valleys obtained with pulsatile delivery, making it easier to maintain therapeutic doses without causing serious side effects that may result from peak serum levels.

A fentanyl transdermal system described in U. S. Pat. No. 4,588,580 that provides continuous systemic delivery of fentanyl for 72 hours is available under various trade designations including the terms "DURAGESIC" and "DUROGESIC". The "DURAGESIC" reservoir transdermal fentanyl patch, sold by Johnson & Johnson, has strengths of 12.5, 25, 50, 75, and 100 $\mu$g/hr patches, which have a total content of fentanyl of 1.25, 2.5, 5.0, 7.5, and 10.0 mg per patch, respectively. The liquid reservoir contains alcohol, a gelling agent, and fentanyl. Reservoir patches are typically larger, bulkier, and more expensive to make than drug-in-adhesive patches. There is also a potential for leakage from a liquid fentanyl reservoir. Drug-in-adhesive patches having fentanyl in acrylate adhesives have been described in U.S. Pat. Pub. Nos. 2002/0119187 (Cantor et al.), 2004/0213832 (Venkatraman et al.), 2006/0039960 (Cordes et al.), and 2004/0001882 (Tisa-Bostedt et al.), and Int. App. Pub. No. 2003/097008 (Stefano et al.). The "DURAGESIC" or "DUROGESIC" matrix transdermal fentanyl patch (also branded as DUROGESIC MAT, DUROGESIC D-TRANS, DUROGESIC SMAT), sold by Johnson & Johnson, has strengths of 12.5, 25, 50, 75, and 100 $\mu$g/hr patches, which have a total content of fentanyl of 2.1, 4.2, 8.4, 12.6, and 16.8 mg per patch, respectively.

### Summary

The present invention is defined in the claims.

In one aspect, the present disclosure provides a transdermal drug delivery device including a backing and an adhesive composition disposed on the backing. The adhesive composition includes a copolymer having at least 50 percent by weight $C_4$ to $C_{10}$ alkyl acrylate units, based on the total weight of the copolymer, and one or more second monomer units selected from the group consisting of vinyl acetate, acrylamide, ethyl acrylate, methyl acrylate, and N-vinyl-2-pyrrolidone. The $C_4$ to $C_{10}$ alkyl acrylate and the one or more second monomer units together make up at least 98 percent by weight of the copolymer. The adhesive composition further includes a skin permeation enhancer in a range from 5 percent to 25 percent by weight, based on the total weight of the adhesive composition, and fentanyl. The adhesive composition is substantially free of undissolved fentanyl.

It has been found that the aforementioned transdermal drug delivery device can match, at least on an in vitro basis, the delivery profile and efficiency performance of the "DURAGESIC" or "DUROGESIC" matrix transdermal fentanyl patch.

It has further been found that a transdermal drug delivery device according to the present disclosure having a lower drug content (e.g., lower coating weight or lower weight percentage of fentanyl) typically delivers fentanyl more efficiently than patches having a higher drug content. Accordingly, the fentanyl is present in a range from 3 percent to 7.5 percent by weight, based on the total weight of the adhesive composition, and the adhesive composition is disposed on the backing in a layer having a coating weight in a range from 3 to 6 mg/cm$^2$. The fentanyl content in the transdermal drug delivery device is up to 0.3 milligrams per square centimeter, and in some embodiments the transdermal drug delivery device has a normalized cumulative flux after 72 hours of at least 600 micrograms per milligram of fentanyl. Thus, the transdermal drug delivery device is capable, in some embodiments, of delivering the same amount of or more fentanyl than certain competitive products having more drug in the patch.

In another aspect, the present disclosure allows treatment of a condition capable of treatment by fentanyl in a mammal. The method includes placing the transdermal drug delivery device as described in any of the above embodiments on the mammal's skin so that the adhesive composition is in contact with the mammal's skin. The method further includes allowing the adhesive composition to remain on the skin for a time sufficient to establish or maintain a therapeutically effective blood level of fentanyl in the mammal.

The terms "comprises" and variations thereof do not have a limiting meaning where these terms appear in the description and claims.

The term "acrylate" encompasses acrylates and methacrylates.

As used herein, "a", "an", "the", "at least one", and "one or more" are used interchangeably.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the description, guidance is provided through lists of examples, which examples can be used in various combinations. In each instance, the recited list serves only as a representative group and should not be interpreted as an exclusive list.

**Detailed Description**

Several subsaturated drug-in-adhesive (DIA) patches including fentanyl have been introduced. In order to match the extended delivery of the transdermal fentanyl reservoir patch sold by Johnson & Johnson under the trade designation "DURAGESIC", however, these patches have contained anywhere from 60% to 140% more drug for a given patch strength than the original reservoir patch. Fentanyl is an expensive drug, so it is desirable to minimize the content. In addition, the US FDA has refused to approve generic-equivalent DIA patches having significantly higher contents than the reservoir patch sold by Johnson & Johnson under the trade designation "DURAGESIC" due to concerns over potential drug abuse.

A transdermal drug delivery device including an acrylate adhesive composition that typically matches, at least on an in vitro basis, the delivery profile and efficiency performance of the transdermal fentanyl reservoir patch sold by Johnson & Johnson under the trade designation "DURAGESIC" has now been found.

The adhesive composition in the transdermal drug delivery device according to the present disclosure includes a copolymer comprising at least 50 percent by weight of $C_4$ to $C_{10}$ alkyl acrylate units, based on the total weight of the copolymer. In some embodiments, the adhesive composition includes a copolymer comprising at least 50 percent by weight of $C_6$ to $C_8$ alkyl acrylate units, in some embodiments, comprising at least 50 percent by weight $C_8$ alkyl acrylate units, based on the total weight of the copolymer. The $C_4$ to $C_{10}$ alkyl acrylate units arise from one or more monomers selected from the group consisting of alkyl acrylates containing 4 to 10, 6 to 8, or 8 carbon atoms in the alkyl group and alkyl methacrylates containing 4 to 10, 6 to 8, or 8 carbon atoms in the alkyl group. Examples of suitable alkyl acrylates and methacrylates include n-butyl, n-pentyl, n-hexyl, isoheptyl, n-nonyl, n-decyl, isohexyl, 2-ethyloctyl, isooctyl and 2-ethylhexyl acrylates and methacrylates. In some embodiments, the alkyl acrylate units comprise at least one of isooctyl acrylate, 2-ethylhexyl acrylate, n-butyl acrylate, or cyclohexyl acrylate units. In some embodiments, the alkyl acrylate units comprise at least one of isooctyl acrylate units or 2-ethylhexyl acrylate units. In some embodiments, the alkyl acrylate units comprise isooctyl acrylate units. In some embodiments, the alkyl acrylate units comprise 2-ethylhexyl acrylate units.

Suitable copolymers for use in the adhesive composition comprise, in some embodiments, about 50 to about 97 percent by weight, in some embodiments, about 60 to about 97 percent by weight, about 50 to about 75 percent by weight, or about 60 to about 75 percent by weight of the $C_4$ to $C_{10}$ alkyl acrylate units, based on the total weight of all monomer units in the copolymer.

Suitable copolymers for use in the adhesive composition comprise, in some embodiments, about 50 to about 97 percent by weight, in some embodiments, about 60 to about 97 percent by weight, about 50 to about 75 percent by weight, or about 60 to about 75 percent by weight of the $C_6$ to $C_8$ alkyl acrylate units, based on the total weight of all monomer units in the copolymer.

Suitable copolymers for use in the adhesive composition comprise, in some embodiments, about 50 to about 97 percent by weight, in some embodiments, about 60 to about 97 percent by weight, about 50 to about 75 percent by weight, or about 60 to about 75 percent by weight of the $C_8$ alkyl acrylate units, based on the total weight of all monomer units in the copolymer.

The acrylate copolymer further comprises second monomer units, which may be considered reinforcing monomer units by those skilled in the art. Reinforcing monomer units typically provide reinforcement to the adhesive composition to prevent it from splitting and oozing during use. Reinforcing monomers can function by increasing the glass transition temperature of the copolymer, causing intermolecular interactions between individual copolymers, covalently crosslinking the copolymer and/or physically crosslinking the copolymer. Second monomers useful for practicing the present disclosure advantageously increase the glass transition temperature of the copolymer. The one or more second monomer units are selected from the group consisting of vinyl acetate, acrylamide, ethyl acrylate, methyl acrylate, and N-vinyl-2-pyrrolidone. In some embodiments, the copolymer comprises at least one of vinyl acetate or acrylamide units. In some embodiments, the copolymer comprises vinyl acetate units.

The $C_4$ to $C_{10}$ alkyl acrylate units and the second monomer units together make up at least about 98% by weight of the copolymer. In some embodiments, the $C_4$ to $C_{10}$ alkyl acrylate units and the second monomer units together make up at least about 98.5%, 99%, or 99.5% by weight of the copolymer. Useful individual amounts of the $C_4$ to $C_{10}$ alkyl acrylate units and the second monomer units vary depending on the selection of the second monomer units.

In some embodiments, the $C_6$ to $C_8$ alkyl acrylate units and the second monomer units together make up at least about 98% by weight of the copolymer. In some embodiments, the $C_6$ to $C_8$ alkyl acrylate units and the second monomer units together make up at least about 98.5%, 99%, or 99.5% by weight of the copolymer. Useful individual amounts of the $C_6$ to $C_8$ alkyl acrylate units and the second monomer units vary depending on the selection of the second monomer units.

In some embodiments, the $C_8$ alkyl acrylate units and the second monomer units together make up at least about 98% by weight of the copolymer. In some embodiments, the $C_8$ alkyl acrylate units and the second monomer units together make up at least about 98.5%, 99%, or 99.5% by weight of the copolymer. Useful individual amounts of the $C_8$ alkyl acrylate units and the second monomer units vary depending on the selection of the second monomer units.

For example, in some embodiments, vinyl acetate can be useful in an amount up to 50% by weight, based on the total weight of the copolymer. In some embodiments, vinyl acetate is present in a range from 5 to 50, 5 to 30, 25 to 50, or 35 to 50 percent by weight, based on the total weight of the copolymer. In some embodiments, the copolymer comprises the $C_4$ to $C_{10}$ alkyl acrylate in a range from 50 to 75 percent by weight and vinyl acetate in a range from 25 to 50 percent by weight, wherein each percent by weight is based on the total weight of the copolymer.

In some embodiments, the copolymer comprises the $C_6$ to $C_8$ alkyl acrylate in a range from 50 to 75 percent by weight and vinyl acetate in a range from 25 to 50 percent by weight, wherein each percent by weight is based on the total weight of the copolymer. In some embodiments, vinyl acetate is present in a range from 5 to 50, 5 to 30, 25 to 50, or 35 to 50 percent by weight, based on the total weight of the copolymer of the $C_6$ to $C_8$ alkyl acrylate and vinyl acetate. In some embodiments, vinyl acetate is present in an amount up to 50 percent by weight, based on the total weight of the copolymer of the $C_6$ to $C_8$ alkyl acrylate and vinyl acetate.

In some embodiments, the copolymer comprises the $C_8$ alkyl acrylate in a range from 50 to 75 percent by weight and vinyl acetate in a range from 25 to 50 percent by weight, wherein each percent by weight is based on the total weight of the copolymer. In some embodiments, vinyl acetate is present in a range from 5 to 50, 5 to 30, 25 to 50, or 35 to 50 percent by weight, based on the total weight of the copolymer of the $C_8$ alkyl acrylate and vinyl acetate. In some embodiments, vinyl acetate is present in an amount up to 50 percent by weight, based on the total weight of the copolymer of the $C_8$ alkyl acrylate and vinyl acetate.

Acrylamide and N-vinyl-2-pyrrolidone tend to stiffen the copolymer more than vinyl acetate. According, a lower amount of these reinforcing monomer units is typically useful. In some embodiments, at least one of N-vinyl-2-pyrrolidone or acrylamide is present in a range from 3 to 10, 5 to 10, or 4 to 8 percent by weight, based on the total weight of the copolymer. If at least one of N-vinyl-2-pyrrolidone or acrylamide is present in less than 3 percent by weight as the only second monomer unit, the amount is typically not enough to prevent the adhesive composition from splitting and oozing during use. If at least one of N-vinyl-2-pyrrolidone or acrylamide is present in more than 10 percent by weight, the adhesive composition is typically too stiff.

Methyl acrylate and ethyl acrylate also tend to stiffen the copolymer more than vinyl acetate. In some embodiments, at least one of ethyl acrylate or methyl acrylate is present in a range from 5 to 25, 5 to 20, 5 to 15, or 10 to 20 percent by weight, based on the total weight of the copolymer.

In some embodiments, the copolymer comprises the $C_4$ to $C_{10}$ alkyl acrylate in a range from 60 to 97 percent by weight, at least one of acrylamide or N-vinyl-2-pyrrolidone in a range from 3 to 10 percent by weight, and vinyl acetate in a range from 0 to 30 percent by weight, wherein each percent by weight is based on the total weight of the copolymer. In some embodiments, the copolymer comprises the $C_4$ to $C_{10}$ alkyl acrylate in a range from 50 to 95 percent by weight, at least one of methyl acrylate or ethyl acrylate in a range from 5 to 20 percent by weight, and vinyl acetate in a range from 0 to 30 percent by weight, wherein each percent by weight is based on the total weight of the copolymer.

In some embodiments, the copolymer comprises the $C_6$ to $C_8$ alkyl acrylate in a range from 60 to 97 percent by weight, at least one of acrylamide or N-vinyl-2-pyrrolidone in a range from 3 to 10 percent by weight, and vinyl acetate in a range from 0 to 30 percent by weight, wherein each percent by weight is based on the total weight of the copolymer. In some embodiments, the copolymer comprises the $C_6$ to $C_8$ alkyl acrylate in a range from 50 to 95 percent by weight, at least one of methyl acrylate or ethyl acrylate in a range from 5 to 20 percent by weight, and vinyl acetate in a range from 0 to 30 percent by weight, wherein each percent by weight is based on the total weight of the copolymer.

In some embodiments, the copolymer comprises the $C_8$ alkyl acrylate in a range from 60 to 97 percent by weight, at least one of acrylamide or N-vinyl-2-pyrrolidone in a range from 3 to 10 percent by weight, and vinyl acetate in a range from 0 to 30 percent by weight, wherein each percent by weight is based on the total weight of the copolymer. In some embodiments, the copolymer comprises the $C_8$ alkyl acrylate in a range from 50 to 95 percent by weight, at least one of methyl acrylate or ethyl acrylate in a range from 5 to 20 percent by weight, and vinyl acetate in a range from 0 to 30 percent by weight, wherein each percent by weight is based on the total weight of the copolymer.

In some embodiments, the copolymer includes less than 2 (in some embodiments, less than 1) percent by weight of hydroxyl-substituted monomer units (e.g., hydroxyethyl acrylate).

Useful copolymer compositions may optionally further comprise a substantially linear macromonomer copolymerizable with the $C_4$ to $C_{10}$ alkyl acrylate and reinforcing monomers and having a weight average molecular weight in the range of about 500 to about 500,000, about 2,000 to about 100,000, or about 4,000 to about 20,000 grams per mole. The macromonomer, when used, is generally present in an amount of not more than about 20% and preferably not more than about 10% by weight based on the total weight of all monomers in the copolymer. Suitable macromonomers include functionally terminated polymethylmethacrylate, styrene/acrylonitrile, polyether, and polystyrene macromonomers. Examples of useful macromonomers and their preparation are described in U.S. Patent No. 4,693,776 (Krampe et al.). In some embodiments, the macromonomer is a polymethylmethacrylate macromonomer.

The copolymers described above can be prepared by any suitable method, for example, that described in U.S. Patent No. RE 24,906 (Ulrich), U.S. Patent No. 4,732,808 (Krampe), and/or U.S. Patent No. 7,097,853 (Garbe).

The inherent viscosity of the copolymer is such as to ultimately provide a suitable pressure sensitive adhesive when used in a device according to the present disclosure. In some embodiments, the copolymer has an inherent viscosity in the range of about 0.2 dL/g to about 2.0 dL/g or about 0.3 dL/g to about 1.4 dL/g. Inherent viscosity may be measured as described in U.S. Patent No. 7,097,853 (Garbe).

Fentanyl is present in the adhesive composition in an amount between about 3% and about 7.5% by weight, based on the total weight of the adhesive composition. In some embodiments, fentanyl is present in the adhesive composition in an amount between about 3% and about 7% by weight, 4% to 7.5% by weight, or 5% to 7% by weight, based on the total weight of the composition. The adhesive composition is substantially free of undissolved fentanyl. The fentanyl is typically completely dissolved in the adhesive composition. The presence of undissolved fentanyl may be detected by examination with an optical microscope at 20x magnification. Having undissolved fentanyl in the adhesive composition may lead to physical instability of the adhesive composition over time and therefore is typically undesirable. The particular amount of fentanyl in the composition that will deliver sufficient fentanyl to achieve a desired therapeutic result varies according to the condition being treated, any drugs being coadministered with the fentanyl, desired duration of treatment, the surface area and location of the skin over which the device is to be placed, and the selection of adjuvant and other components of the transdermal delivery device.

If desired, the adhesive composition can contain components that modify the properties of the copolymer, such as plasticizers or tackifiers, in amounts readily determinable to those of skill in the art.

An adhesive composition useful in the drug delivery device according to the present disclosure includes a skin permeation enhancer. A variety of skin permeation enhancers may be useful. Examples of suitable skin permeation enhancers include $C_8$-$C_{36}$ fatty alcohols such as oleyl alcohol and lauryl alcohol; lower alkyl esters of $C_8$-$C_{36}$ fatty acids such as ethyl oleate, isopropyl myristate, butyl stearate, and methyl laurate; tetraglycol (tetrahydrofurfuryl alcohol polyethylene glycol ether); and propylene glycol; and combinations of any of these. In some embodiments, the skin permeation enhancer comprises at least one of isopropyl myristate, tetraglycol, methyl laurate, propylene glycol, propylene glycol monolaurate, ethyl oleate, isopropyl myristate, 2-octyl-1-dodecanol, lauryl lactate, lauryl alcohol, and combinations of any of these. In some embodiments, the skin permeation enhancer is methyl laurate.

In an adhesive composition useful in the drug delivery device according to the present disclosure, the skin permeation enhancer(s) is either dispersed, typically substantially uniformly, or dissolved in the adhesive composition and is present in an amount that enhances fentanyl permeation through the skin compared to a like composition not containing a skin permeation enhancer when this phenomenon is measured using the skin permeation model described below. The amount of the skin permeation enhancer also typically affects the physical properties in a transdermal drug delivery device. For example, it is desirable to have sufficiently little cold flow that a device of the invention is stable to flow upon storage. It is also desirable that it adhere well to the skin and release cleanly from the skin. In order to achieve resistance to cold flow, skin adhesion and clean release, the amount and structure of the comonomers in the copolymer, the inherent viscosity of the copolymer, and the amount and type of adjuvant are selected such that the adhesive layer(s) obtain the desired balance of these properties.

The total amount of skin penneation enhancer is from about 5% to about 25% by weight based on the total weight of the adhesive composition. If the skin permeation enhancer is present in less than 5% by weight, it may not be effective for enhancing fentanyl permeation through the skin. If the skin permeation enhancer is present in greater than 25% by weight, the adhesive composition may be too soft and leave residue on the skin. In some embodiments, the adhesive composition comprises the skin permeation enhancer in a range from 10% to 23%, 10% to 20%, 13% to 20%, or 15% to 20% by weight, based on the total weight of the adhesive composition.

In some embodiments, the adhesive composition will have a shear creep compliance (as determined by the test method below) of between about $1.0 \times 10^{-5}$ and $5.0 \times 10^{-5}$ cm$^2$/dyne. Adhesive compositions in this range have good conformance and adhesion to skin, while not being so soft as to leave excessive residue on the skin. In some embodiments, the shear creep compliance will be between $1.0 \times 10^{-5}$ and $4.0 \times 10^{-5}$ cm$^2$/dyne, $1.5 \times 10^{-5}$ and $4.0 \times 10^{-5}$ cm2/dyne, or $1.5 \times 10^{-5}$ and $3.0 \times 10^{-5}$ cm$^2$/dyne.

In some embodiments, the transdermal drug delivery device is bioequivent to a transdermal fentanyl reservoir patch obtained from Johnson & Johnson under the trade designation "DURAGESIC". That is, the drug delivery device according to the present disclosure has a delivery profile that generally matches that of the transdermal fentanyl reservoir patch obtained from Johnson & Johnson under the trade designation "DURAGESIC". In addition, in some embodiments the transdermal drug delivery device has an efficiency performance that generally matches that of the transdermal fentanyl reservoir patch obtained from Johnson & Johnson under the trade designation "DURAGESIC".

In some embodiments, the transdennal drug delivery device is bioequivent to a transdermal fentanyl matrix patch obtained from Johnson & Johnson under the trade designation "DURAGESIC". That is, the drug delivery device according to the present disclosure has a delivery profile that generally matches that of the transdermal fentanyl reservoir patch obtained from Johnson & Johnson under the trade designation "DURAGESIC".

Comparison of delivery profile and efficiency performance may be made using two key in vitro parameters: 1) a 'shape' factor that describes the delivery profile and 2) a normalized cumulative flux that describes total flux for a given amount of drug.

The shape factor "S" is described by formula:

$$S = (72 \text{ hours} * \text{peak flux}) / \text{cumulative flux in a 72-hour period},$$

where 72 hours*peak flux is a peak flux during a 72-hour permeation study and is measured in units of micrograms per square centimeter per hour and cumulative flux is measured over the entire 72-hour time period in units of micrograms per square centimeter. If peak flux was maintained during the entire 72-hour period, then the shape factor would be 1.0. Otherwise, the shape factor will be greater than 1, since 72*(peak flux) will be greater than the cumulative flux over the 72-hour period. It is desirable that the shape factor for the drug delivery device according to the present disclosure be similar to the shape factor for the "DURAGESIC" patch (either reservoir or matrix). This similarity in shape is important, since a bioequivalence study needs to match (within an 80 to 125% confidence interval) both the $C_{max}$ (maximum plasma level) and AUC (area under the plasma curve) of the product being compared to. Although $C_{max}$ and AUC can generally be adjusted directly by adjusting patch size, they will change in concert with each other. If the delivery shape is either too peaked or too flat, then it becomes more difficult to simultaneously match both $C_{max}$ and AUC. The shape factor for "DURAGESIC" reservoir was measured as 1.81, by taking averages of flux data from several permeability studies on a number of different lots of cadaver skin according the method described in the Examples, below. Thus, in some embodiments, the shape factor is in a range from 1.3 to 2.2, 1.4 to 1.9, 1.5 to 1.9, or 1.6 to 1.8. To some degree the shape factor is influenced by the relative permeability of the skin, since this will affect how quickly the patch is depleted of drug. Thus the absolute values of shape factors should generally be compared to control samples (e.g., "DURAGESIC" reservoir) tested on the same lot(s) of skin.

The normalized cumulative flux is the cumulative flux per mg fentanyl (or alternatively, per 2.5 mg fentanyl, which corresponds to the content in the 25 $\mu$g/hr "DURAGESIC" reservoir patch). If the normalized cumulative flux of the drug delivery device according to the present disclosure is greater than the normalized cumulative flux of the "DURAGESIC" reservoir patch, then the experimental patch can be expected to deliver as much or more fentanyl from a patch with equivalent total content to "DURAGESIC" reservoir patch. Other bioequivalent commercial acrylate patches have a considerably lower normalized cumulative flux than that of the "DURAGESIC" reservoir patch. That is, they need an excess of drug when compared to the "DURAGESIC" reservoir patch to match bioequivalent delivery. The normalized cumulative flux of the "DURAGESIC" reservoir patch was found to be 1681 $\mu$g/(2.5 mg fentanyl), by taking averages of flux data from several permeability studies on a number of different lots of cadaver skin according to the test method described in the Examples, below. Accordingly, the normalized cumulative flux of the "DURAGESIC" reservoir patch was found to be 672 $\mu$g/(mg fentanyl). Thus, in some embodiments, a transdermal drug delivery device according to the present disclosure has a normalized cumulative flux after 72 hours of at least 600 micrograms per milligram of fentanyl, in some embodiments, at least 610, 620, 650, or 675 micrograms per milligram of fentanyl.

The higher normal cumulative flux for the transdermal drug delivery device according to the present disclosure provides evidence of its efficiency. The transdermal drug delivery device according to the present disclosure can deliver the same or higher amount of fentanyl through the skin as a commercially available patch using less fentanyl in the drug delivery device. In some reference embodiments, the fentanyl content in the transdermal drug delivery device is up to 0.5, 0.45, 0.4, or 0.35 milligrams per square centimeter and in the invention up to 0.3 milligrams per square centimeter.

In some embodiments, the high efficiency can be unexpectedly achieved by lowering the coating weight of the adhesive composition on the backing. The adhesive composition is disposed on the backing in a layer having a coating weight in a range from 3 mg/cm$^2$ to 6 mg/cm$^2$, preferably 3 mg/cm$^2$ to 5.5 mg/cm$^2$, or 4 mg/cm$^2$ to 6 mg/cm$^2$. The coating weight is the weight of adhesive per unit area. It can be determined by weighing the drug delivery device or a fixed area thereof, which includes the adhesive composition, and subtracting the weight of the backing.

The transdermal delivery devices according to the present disclosure can be made in any useful form. For example, the drug delivery device can be made in the form of a tape, a patch, a sheet, or a dressing. Generally, the device will be in the form of a patch of a size suitable to deliver a preselected amount of fentanyl through the skin. In some embodiments, a 12 μg/hr strength device will have a surface area of about 3 cm$^2$ to about 6 cm$^2$ for a patch. In some embodiments, a 25 μg/hr strength device will have a surface area of about 6 cm$^2$ to about 15 cm$^2$ for a patch, in some embodiments, about 6 cm$^2$ to about 10 cm$^2$. In some embodiments, a 50 μg/hr strength device will have a surface area of about 12 cm$^2$ to about 30 cm$^2$ for a patch, in some embodiments, about 12 cm$^2$ to about 20 cm$^2$. In some embodiments, a 75 μg/hr strength device will have a surface area of about 18 cm$^2$ to about 45 cm$^2$ for a patch, in some embodiments, about 18 cm$^2$ to about 30 cm$^2$. In some embodiments, a 100 μg/hr strength device will have a surface area of about 24 cm$^2$ to about 60 cm$^2$ for a patch, in some embodiments, about 24 cm$^2$ to about 40 cm$^2$. The transdermal drug delivery device according to the present disclosure can have a total content of fentanyl selected from the group consisting of about 1.25 milligrams, about 2.5 milligrams, about 5 milligrams, about 7.5 milligrams, and about 10 milligrams, for example.

A transdermal drug delivery device according to the present disclosure also comprises a backing. The backing is typically flexible such that the device conforms to the skin. The backing may be breathable or occlusive and may comprise at least one of fabric, polymer films, coated paper products, and aluminum films. In some embodiments, the backing is an occlusive backing. Suitable backing materials include conventional flexible backing materials used for pressure sensitive adhesive tapes, such as polyethylene, particularly low density polyethylene, linear low density polyethylene, metallocene polyethylenes, high density polyethylene, polypropylene, polyesters such as polyethylene terephthalate, randomly oriented nylon fibers, ethylene-vinyl acetate copolymer, polyurethane, natural fibers such as rayon and the like. Backings that are multi-layered such as polyethylene terephthalate-aluminum-polyethylene composites are also suitable. The backing is typically substantially inert to the components of the adhesive layer.

Transdermal devices according to the present disclosure may be prepared by combining the copolymer, the skin permeation enhancer, and the fentanyl with an organic solvent (e.g., ethyl acetate, isopropanol, methanol, acetone, 2-butanone, ethanol, toluene, alkanes, and mixtures thereof) to provide a coating composition. The mixture can be shaken or stirred until a homogeneous coating composition is obtained. The resulting composition may then be applied to a release liner using conventional coating methods (e.g., knife coating or extrusion die coating) to provide a predetermined uniform thickness of coating composition. Suitable release liners include conventional release liners comprising a known sheet material such as a polyester web, a polyethylene web, a polystyrene web, or a polyethylene-coated paper coated with a suitable fluoropolymer or silicone based coating. The release liner that has been coated with the composition may then be dried and laminated onto a backing using conventional methods.

A transdermal drug delivery composition of the disclosure can be used to induce an analgesic effect. The present disclosure allows treatment of a condition capable of treatment by fentanyl in a mammal. The method includes placing the transdermal drug delivery device as described in any of the above embodiments on the mammal's skin so that the adhesive composition is in contact with the mammal's skin. The method further includes allowing the adhesive composition to remain on the skin for a time sufficient to establish or maintain a therapeutically effective blood level of fentanyl in the mammal, for example, to maintain the intended analgesic effect. The time that constitutes a sufficient time can be selected by those skilled in the art with consideration of the flux rate provided by the device of the invention and of the condition being treated.

The amount of fentanyl that needs to be delivered and the serum concentrations that are necessary to be therapeutically effective show considerable variation between individuals. A tolerance to fentanyl generally develops with continued use, typically necessitating the need for increased dosages over time of treatment. Because of this inter- and intra-patient variation, a wide range of therapeutically effective fentanyl serum concentrations have been reported. Further details may be found in the monographs "Fentanyl Citrate", AHFS 98 Drug Information, ed.: G. K. McEvoy, American Society of Health-Systems Pharmacists, p. 1677-1683 (1998) and "Fentanyl: A Review for Clinical and Analytical Toxicologists", A. Poklis, Clinical Toxicology, 33(5), 439-447 (1995).

EXAMPLES

Example 1

Fentanyl (0.8400 g) and a 40:60 blend of methanol/ethyl acetate (0.4766 g) were added together and mixed until all of the fentanyl was dissolved to form a fentanyl solution. Methyl laurate (2.6549) and solvated copolymer (28.2867 g of isooctyl acrylate/acrylamide 93:7 copolymer, 31.2% solids, in ethyl acetate/methanol 91:9) were added to the fentanyl solution and mixed until a uniform coating formulation was obtained. The coating formulation was knife coated at a wet thickness of 230 micrometers ($\mu$m) onto a release liner (SCOTCHPAK™ 9742 fluoropolymer coated release liner; available from 3M Company). The coated liner was oven dried for 2 minutes at 43°C followed by 4 minutes at 63°C. The coated liner was laminated onto a backing (SCOTCHPAK™ 9732 polyester film laminate; available from 3M Company) to form a bulk transdermal patch laminate. The nominal fentanyl and methyl laurate concentrations of the dried coating were 7.0% and 20.0%, respectively. The dried adhesive matrix coating weight was 5.5 mg/cm$^2$. Appropriately sized transdermal patches were punched from the bulk laminate for subsequent testing. The permeation through human cadaver skin was determined using the test method described below and the results are reported in Table 1. Adhesive compliance was determined using the test method described below and the results are reported in Table 1.

Examples 2 to 12

Formulations were prepared as in Example 1 with the exception that the nominal fentanyl and methyl laurate concentrations and the nominal dried adhesive matrix coating weight were varied as shown in Table 1. The permeation through human cadaver skin was determined using the test method described below and the results are reported in Table 1. Adhesive compliance (where measured) was determined using the test method described below and the results are reported in Table 1.

Examples 13 - 14

Formulations were prepared as in Examples 11 and 12 with the exception that the solvated copolymer was isooctyl acrylate/acrylamide/vinyl acetate 75:5:20 copolymer, 24.0% solids, in ethyl acetate/methanol 90:10 - note: confirm this ratio with JPD). The permeation through human cadaver skin was determined using the test method described below and the results are reported in Table 1. Adhesive compliance was determined using the test method described below and the results are reported in Table 1.

Example 15

A formulation was prepared according to the general procedure of Example 1 with the exception that the formulation contained light mineral oil NF as a replacement for methyl laurate. The nominal fentanyl and light mineral oil NF concentrations of the dried coating were 7.0% and 10.0%, respectively. The dried adhesive matrix coating weight was 4.6 mg/cm$^2$. The permeation through human cadaver skin was determined using the test method described below and the results are reported in Table 2.

Example 16

A formulation was prepared according to the general procedure of Example 15 with the exception that the nominal fentanyl and light mineral oil NF concentrations of the dried coating were 7.0% and 20.0%, respectively. The dried adhesive matrix coating weight was 5.2 mg/cm$^2$. The permeation through human cadaver skin was determined using the test method described below and the results are reported in Table 2.

Example 17

A formulation was prepared according to the general procedure of Example 1 with the exception that the formulation contained lauryl lactate as a replacement for methyl laurate. The nominal fentanyl and lauryl lactate concentrations of the dried coating were 7.0% and 20.0%, respectively. The dried adhesive matrix coating weight was 5.6 mg/cm$^2$. The permeation through human cadaver skin was determined using the test method described below and the results are reported in Table 2.

Example 18

A formulation was prepared according to the general procedure of Example 1 with the exception that the formulation contained a combination of light mineral oil NF and lauryl lactate as a replacement for methyl laurate. The nominal fentanyl, light mineral oil NF and lauryl lactate concentrations of the dried coating were 7.0%, 10.0% and 10.0%, respectively. The dried adhesive matrix coating weight was 5.3 mg/cm$^2$. The permeation through human cadaver skin was determined using the test method described below and the results are reported in Table 2.

Example 19

A formulation was prepared according to the general procedure of Example 1 with the exception that the formulation also contained light mineral oil NF in addition to methyl laurate. The nominal fentanyl, light mineral oil NF and methyl laurate concentrations of the dried coating were 7.0%, 10.0% and 10.0%, respectively. The dried adhesive matrix coating weight was 4.8 mg/cm$^2$. The permeation through human cadaver skin was determined using the test method described below and the results are reported in Table 2.

Example 20

A formulation was prepared according to the general procedure of Example 1 with the exception that the formulation also contained lauryl lactate in addition to methyl laurate. The nominal fentanyl, lauryl lactate and methyl laurate concentrations of the dried coating were 7.0%, 10.0% and 10.0%, respectively. The dried adhesive matrix coating weight was 5.1 mg/cm$^2$. The permeation through human cadaver skin was determined using the test method described below and the results are reported in Table 2.

Example 21

A formulation was prepared according to the general procedure of Example 1 with the exception that the formulation also contained 2-octyl-1-dodecanol in addition to methyl laurate. The nominal fentanyl, 2-octyl-1-dodecanol and methyl laurate concentrations of the dried coating were 6.6%, 12.0% and 12.0%, respectively. The dried adhesive matrix coating weight was 5.1 mg/cm$^2$. The permeation through human cadaver skin was determined using the test method described below and the results are reported in Table 2.

Example 22

A formulation was prepared according to the general procedure of Example 1 with the exception that the formulation contained a combination of ethyl oleate and propylene glycol as a replacement for methyl laurate. The nominal fentanyl, ethyl oleate and propylene glycol concentrations of the dried coating were 6.3%, 12.0% and 12.0%, respectively. The dried adhesive matrix coating weight was 4.2 mg/cm$^2$. The permeation through human cadaver skin was determined using the test method described below and the results are reported in Table 2.

Example 23

A formulation was prepared according to the general procedure of Example 1 with the exception that the formulation contained a combination of ethyl oleate and light mineral oil NF as a replacement for methyl laurate. The nominal fentanyl, ethyl oleate and light mineral oil NF concentrations of the dried coating were 4.7%, 12.0% and 12.0%, respectively. The dried adhesive matrix coating weight was 4.3 mg/cm$^2$. The permeation through human cadaver skin was determined using the test method described below and the results are reported in Table 2.

Example 24

A formulation was prepared according to the general procedure of Example 1 with the exception that the formulation contained a combination of ethyl oleate and isopropyl myristate as a replacement for methyl laurate. The nominal fentanyl, ethyl oleate and isopropyl myristate concentrations of the dried coating were 5.3%, 12.0% and 12.0%, respectively. The dried adhesive matrix coating weight was 4.6 mg/cm$^2$. The permeation through human cadaver skin was determined using the test method described below and the results are reported in Table 2.

Example 25

A formulation was prepared according to the general procedure of Example 1 with the exception that the formulation contained a combination of propylene glycol monolaurate and light mineral oil NF as a replacement for methyl laurate. The nominal fentanyl, propylene glycol monolaurate and light mineral oil NF concentrations of the dried coating were 7.0%, 12.0% and 12.0%, respectively. The dried adhesive matrix coating weight was 4.6 mg/cm$^2$. The permeation through human cadaver skin was determined using the test method described below and the results are reported in Table 2.

Example 26

A formulation was prepared according to the general procedure of Example 1 with the exception that the formulation contained a combination of 2-octyl-1-dodecanol and light mineral oil NF as a replacement for methyl laurate. The nominal fentanyl, 2-octyl-1-dodecanol and light mineral oil NF concentrations of the dried coating were 5.4%, 12.0% and 12.0%, respectively. The dried adhesive matrix coating weight was 4.7 mg/cm$^2$. The permeation through human cadaver skin was determined using the test method described below and the results are reported in Table 2.

In Vitro Skin Permeation Test Method

The skin permeation data given in the examples above was obtained using the following test method. The release liner was removed from a 1.0 cm$^2$ patch and the patch was applied to human cadaver skin and pressed to cause uniform contact with the skin. The resulting patch/skin laminate was placed patch side up across the orifice of the lower portion of a vertical diffusion cell. The diffusion cell was assembled and the lower portion filled with 5 mL of warm (32°C) receptor fluid (0.1 M phosphate buffer, pH 6.5) so that the receptor fluid contacted the skin. The sampling port was covered except when in use.

The cells were maintained at 32 ± 2°C throughout the course of the experiment. The receptor fluid was stirred by means of a magnetic stirrer throughout the experiment to assure a uniform sample and a reduced diffusion barrier on the dermal side of the skin. The entire volume of receptor fluid was withdrawn at specified time intervals and immediately replaced with fresh fluid. The withdrawn fluid was filtered through a 0.45 μm filter. Approximately 1 to 2 mL were then analyzed for fentanyl using conventional high performance liquid chromatography methods (Column: 80A Extend C18 4.6mm x 75mm, 3.5μm particle size; Mobile phase: 35/65 25mM ammonium hydroxide/acrylonitrile. Flow Rate: 1.5 mL/min; Detector: UV at 210 nm; Injection Volume: 25 μL; Run time: 3.0 minutes). The cumulative amount of fentanyl penetrating through the skin at each time interval was calculated and reported as μg/cm$^2$. A "normalized" cumulative flux, CFnorm, was determined by calculating the cumulative flux at 72 hours that would be achieved from a patch sized so as to have a content of 2.5 mg of fentanyl (CFnorm = (Cum. flux at 72 hrs in μg/cm$^2$)*(2.5 mg)/(Content in mg/cm$^2$)). A shape factor, S, was determined as follows: S = (72 * Peak flux)/(Cum. flux at 72 hours). The shape factor, S, is indicative of how much higher the peak flux is as compared to the average flux over the entire time period.

Adhesive Compliance Test Method

The release liner is removed from a sample of the material to be tested. The exposed adhesive surface is folded back on itself in the lengthwise direction to produce a "sandwich" configuration, i.e., backing/adhesive/backing. The "sandwiched" sample is passed through a laminator, or alternatively rolled with a hand-operated roller, then two 5 cm$^2$ test samples are cut using a circular die. One test sample is centered on a first stationary plate of a parallel plate shear-creep rheometer. The small, non-stationary plate of the shear-creep rheometer is centered over the first sample on the first stationary plate such that the string attaching the weight (500 g) is toward the front of the rheometer. The second test sample is centered on the upper surface of the small, non-stationary plate. A second stationary plate is placed over the second test sample and the entire assembly is clamped into place to prevent slippage of the stationary plates. The plates are placed in a horizontal configuration. The end of the small, non-stationary plate that is opposite the end with the string and weight is monitored by a displacement measurement mechanism. The string is extended over the front pulley of the rheometer, but the weight is initially supported so that it does not exert force on the non-stationary plate. The support for the weight is removed so that the weight hangs free and the displacement of the non-stationary plate is measured for 3 minutes. The displacement at 3 minutes is used to calculate compliance, J, using the equation:

$$J = 2*A*X/(h*f)$$

where A is the area of one face of the test sample, h is the thickness of the adhesive mass (i.e., two times the matrix thickness of the sample being tested), X is the displacement and f is the force due to the mass attached to the string. All testing is performed at 22°C ± 1 °C.

Table 1.

| Ex. | Fentanyl Wt. % | Methyl laurate Wt. % | Coating Weight (mg/cm$^2$) | Cum Flux 12 hrs (ug/cm$^2$) | Cum Flux 24 hrs (ug/cm$^2$) | Cum Flux 48 hrs (ug/cm$^2$) | Cum Flux 72 hrs (ug/cm$^2$) | CFnorm | S | Compliance [x 10$^{-5}$ cm$^2$/dyne] |
|---|---|---|---|---|---|---|---|---|---|---|
| 1 | 7 | 20 | 5.5 | 53 | 118 | 201 | 251 | 1631 | 1.54 | 3.04 |
| 2 | 7 | 10 | 3.5 | 33 | 72 | 110 | 140 | 1423 | 1.64 | |
| 3 | 7 | 10 | 5.5 | 32 | 87 | 155 | 204 | 1326 | 1.53 | |
| 4 | 7 | 15 | 3.5 | 54 | 109 | 167 | 199 | 2029 | 1.75 | |
| 5 | 7 | 15 | 5.5 | 48 | 119 | 201 | 253 | 1643 | 1.67 | |
| 6 | 7 | 20 | 3.5 | 63 | 118 | 175 | 205 | 2092 | 1.96 | |
| 7 | 7 | 25 | 3.5 | 61 | 119 | 171 | 195 | 1986 | 2.05 | |
| 8 | 7 | 25 | 5.5 | 57 | 119 | 197 | 243 | 1580 | 1.6 | 4.51 |
| 9 | 7.4 | 10 | 5.5 | 30 | 64 | 129 | 178 | 1094 | 1.27 | |
| 10 | 7.4 | 20 | 5.5 | 33 | 76 | 154 | 211 | 1295 | 1.31 | |
| 11 | 6 | 15 | 4.5 | 47 | 87 | 138 | 166 | 1537 | 1.9 | 1.43 |
| 12 | 6 | 20 | 4.5 | 43 | 86 | 144 | 176 | 1626 | 1.78 | 2.81 |
| 13 | 6 | 15 | 4.5 | 44 | 80 | 131 | 164 | 1519 | 2.05 | 2.11 |
| 14 | 6 | 20 | 4.5 | 29 | 54 | 96 | 129 | 1194 | 1.76 | 3.03 |

EP 3 233 081 B1

Table 2.

| Ex. | Cum Flux 12 hrs (ug/cm$^2$) | Cum Flux 24 hrs (ug/cm$^2$) | Cum Flux 48 hrs (ug/cm$^2$) | Cum Flux 72 hrs (ug/cm$^2$) | CFnorm | S |
|---|---|---|---|---|---|---|
| 15 | 28 | 68 | 122 | 155 | 1215 | 1.55 |
| 16 | 33 | 90 | 178 | 238 | 1635 | 1.42 |
| 17 | 38 | 90 | 162 | 206 | 1326 | 1.49 |
| 18 | 25 | 68 | 139 | 192 | 1285 | 1.33 |
| 19 | 33 | 88 | 172 | 229 | 1697 | 1.44 |
| 20 | 39 | 90 | 167 | 219 | 1545 | 1.51 |
| 21 | 39 | 70 | 115 | 144 | 1282 | 1.93 |
| 22 | 40 | 70 | 111 | 138 | 1331 | 2.04 |
| 23 | 34 | 58 | 94 | 116 | 1428 | 1.88 |
| 24 | 34 | 63 | 107 | 133 | 1367 | 1.79 |
| 25 | 45 | 83 | 140 | 175 | 1364 | 1.76 |
| 26 | 26 | 47 | 82 | 106 | 1041 | 1.69 |

**Claims**

1. A transdermal drug delivery device, comprising

   a backing; and
   an adhesive composition disposed on the backing, the adhesive composition comprising:

   a copolymer comprising at least 50 percent by weight $C_4$ to $C_{10}$ alkyl acrylate units, based on the total weight of the copolymer and one or more second monomer units selected from the group consisting of vinyl acetate, acrylamide, ethyl acrylate, methyl acrylate, and N-vinyl-2-pyrrolidone, wherein the $C_4$ to $C_{10}$ alkyl acrylate and the one or more second monomer units together make up at least 98 percent by weight of the copolymer;
   a skin permeation enhancer in a range from 5 percent to 25 percent by weight, based on the total weight of the adhesive composition; and
   fentanyl in a range from 3 percent to 7.5 percent by weight, based on the total weight of the adhesive composition,

   wherein the adhesive composition is substantially free of undissolved fentanyl, and wherein the adhesive composition is disposed on the backing in a layer having a coating weight in a range from 3 to 6 mg/cm$^2$, and wherein the fentanyl content in the transdermal drug delivery device is up to 0.3 milligrams per square centimeter.

2. The transdermal drug delivery device of claim 1, further comprising a total content of fentanyl selected from the group consisting of about 1.25 milligrams, about 2.5 milligrams, about 5 milligrams, about 7.5 milligrams, and about 10 milligrams.

3. The transdermal drug delivery device of claim 1 or 2, wherein the copolymer comprises at least two of the second monomer units, and wherein the $C_4$ to $C_{10}$ alkyl acrylate and the at least two of the second monomer units together make up at least 98 percent by weight of the copolymer.

4. The transdermal drug delivery device of any preceding claim, wherein the copolymer comprises the $C_4$ to $C_{10}$ alkyl acrylate in a range from 60 to 97 percent by weight, at least one of acrylamide or N-vinyl-2-pyrrolidone in a range from 3 to 10 percent by weight, and vinyl acetate in a range from 0 to 30 percent by weight, wherein each percent by weight is based on the total weight of the copolymer.

5. The transdermal drug delivery device of claim 1 or 2, wherein the copolymer comprises the $C_4$ to $C_{10}$ alkyl acrylate in a range from 50 to 75 percent by weight and vinyl acetate in a range from 25 to 50 percent by weight, wherein each percent by weight is based on the total weight of the copolymer.

6. The transdermal drug delivery device of claim 4 or 5, wherein the $C_4$ to $C_{10}$ alkyl acrylate is isooctyl acrylate, 2-ethylhexyl acrylate, or a combination thereof.

7. The transdermal drug delivery device of any preceding claim, wherein the adhesive composition comprises skin permeation enhancer in a range from 13 to 20 percent by weight, based on the total weight of the adhesive composition.

8. The transdermal drug delivery device of any preceding claim, wherein the skin permeation enhancer comprises at least one of isopropyl myristate, tetraglycol, methyl laurate, propylene glycol, propylene glycol monolaurate, ethyl oleate, 2-octyl-1-dodecanol, lauryl lactate, or lauryl alcohol.

9. The transdermal drug delivery device of any preceding claim for use in the treatment of a condition capable of treatment by fentanyl in a mammal, wherein the transdermal drug delivery device is to be employed in a method comprising:

placing the transdermal drug delivery device on the mammal's skin so that the adhesive composition is in contact with the mammal's skin; and
allowing the adhesive composition to remain on the skin for a time sufficient to establish or maintain a therapeutically effective blood level of fentanyl in the mammal.

## Patentansprüche

1. Eine transdermale Arzneimittelverabreichungsvorrichtung, umfassend

einen Träger; und
eine Klebstoffzusammensetzung, welche auf dem Träger angeordnet ist, wobei die Klebstoffzusammensetzung umfasst:

ein Copolymer, umfassend mindestens 50 Gew.-% an $C_4$- bis $C_{10}$-Alkylacrylat-Einheiten, bezogen auf das Gesamtgewicht des Copolymers, und eine oder mehrere zweite Monomereinheiten, ausgewählt aus der Gruppe bestehend aus Vinylacetat, Acrylamid, Ethylacrylat, Methylacrylat und N-Vinyl-2-pyrrolidon, wobei das $C_4$- bis $C_{10}$-Alkylacrylat und die eine oder mehreren zweiten Monomereinheiten zusammen mindestens 98 Gew.-% des Copolymers ausmachen;
einen Hautpermeationsverstärker in einem Bereich von 5 Gew.-% bis 25 Gew.-%, bezogen auf das Gesamtgewicht der Klebstoffzusammensetzung; und
Fentanyl in einem Bereich von 3 Gew.-% bis 7,5 Gew.-%, bezogen auf das Gesamtgewicht der Klebstoffzusammensetzung,

wobei die Klebstoffzusammensetzung im Wesentlichen frei von ungelöstem Fentanyl ist und wobei die Klebstoffzusammensetzung auf dem Träger in einer Schicht aufgebracht ist, welche ein Beschichtungsgewicht in einem Bereich von 3 bis 6 mg/cm$^2$ aufweist, und
wobei der Fentanylgehalt in der transdermalen Arzneimittelverabreichungsvorrichtung bis zu 0,3 mg pro cm$^2$ beträgt.

2. Die transdermale Arzneimittelverabreichungsvorrichtung gemäß Anspruch 1, ferner umfassend einen Gesamtgehalt an Fentanyl ausgewählt aus der Gruppe bestehend aus etwa 1,25 mg, etwa 2,5 mg, etwa 5 mg, etwa 7,5 mg und etwa 10 mg.

3. Die transdermale Arzneimittelverabreichungsvorrichtung gemäß Anspruch 1 oder 2, wobei das Copolymer mindestens zwei der zweiten Monomereinheiten umfasst und wobei das $C_4$- bis $C_{10}$-Alkylacrylat und die mindestens zwei der zweiten Monomereinheiten zusammen mindestens 98 Gew.-% des Copolymers ausmachen.

4. Die transdermale Arzneimittelverabreichungsvorrichtung gemäß einem der vorhergehenden Ansprüche, wobei das

Copolymer das $C_4$- bis $C_{10}$-Alkylacrylat in einem Bereich von 60 bis 97 Gew.-%, mindestens eines von Acrylamid oder N-Vinyl-2-pyrrolidon in einem Bereich von 3 bis 10 Gew.-%, und Vinylacetat in einem Bereich von 0 bis 30 Gew.-% umfasst, wobei jedes Gew.-% auf das Gesamtgewicht des Copolymers bezogen ist.

5. Die transdermale Arzneimittelverabreichungsvorrichtung gemäß Anspruch 1 oder 2, wobei das Copolymer das $C_4$- bis $C_{10}$-Alkylacrylat in einem Bereich von 50 bis 75 Gew.-% und Vinylacetat in einem Bereich von 25 bis 50 Gew.-% umfasst, wobei jedes Gew.-% auf das Gesamtgewicht des Copolymers bezogen ist.

6. Die transdermale Arzneimittelverabreichungsvorrichtung gemäß Anspruch 4 oder 5, wobei das $C_4$- bis $C_{10}$-Alkylacrylat Isooctylacrylat, 2-Ethylhexylacrylat oder eine Kombination davon ist.

7. Die transdermale Arzneimittelverabreichungsvorrichtung gemäß einem der vorhergehenden Ansprüche, wobei die Klebstoffzusammensetzung einen Hautpermeationsverstärker in einem Bereich von 13 bis 20 Gew.-%, bezogen auf das Gesamtgewicht der Klebstoffzusammensetzung, umfasst.

8. Die transdermale Arzneimittelverabreichungsvorrichtung gemäß einem der vorhergehenden Ansprüche, wobei der Hautpermeationsverstärker mindestens eines von Isopropylmyristat, Tetraglykol, Methyllaurat, Propylenglykol, Propylenglykolmonolaurat, Ethyloleat, 2-Octyl-1-dodecanol, Lauryllactat oder Laurylalkohol umfasst.

9. Die transdermale Arzneimittelverabreichungsvorrichtung gemäß einem der vorhergehenden Ansprüche zur Verwendung bei der Behandlung eines durch Fentanyl behandelbaren Zustandes bei einem Säugetier, wobei die transdermale Arzneimittelverabreichungsvorrichtung in einem Verfahren eingesetzt werden soll, umfassend:

Platzieren der transdermalen Arzneimittelverabreichungsvorrichtung auf der Haut des Säugetiers, so dass die Klebstoffzusammensetzung in Kontakt mit der Haut des Säugetiers ist; und
Verbleibenlassen der Klebstoffzusammensetzung auf der Haut für eine Zeit, welche ausreicht, um einen therapeutisch wirksamen Blutspiegel von Fentanyl in dem Säugetier herzustellen oder aufrechtzuerhalten.

## Revendications

1. Dispositif d'administration transdermique de médicament comprenant

un support ; et
une composition adhésive disposée sur le support, la composition adhésive comprenant :

un copolymère comprenant au moins 50 % en poids d'unités acrylate d'alkyle en $C_4$ à $C_{10}$, par rapport au poids total du copolymère, et une ou plusieurs secondes unités monomères sélectionnées dans le groupe constitué par l'acétate de vinyle, l'acrylamide, l'acrylate d'éthyle, l'acrylate de méthyle, et la N-vinyl-2-pyrrolidone, dans lequel l'acrylate d'alkyle en $C_4$ à $C_{10}$ et la ou les secondes unités monomères constituent ensemble au moins 98 % en poids du copolymère ;
un activateur de perméation cutanée dans une plage de 5 % à 25 % en poids, par rapport au poids total de la composition adhésive ; et
du fentanyl dans une plage de 3 % à 7,5 % en poids, par rapport au poids total de la composition adhésive,

dans lequel la composition adhésive est pratiquement exempte de fentanyl non dissous, et
dans lequel la composition adhésive est disposée sur le support en une couche ayant un poids de revêtement dans une plage de 3 à 6 mg/cm$^2$, et
dans lequel la teneur en fentanyl dans le dispositif d'administration transdermique de médicament s'étend jusqu'à 0,3 milligramme par centimètre carré.

2. Dispositif d'administration transdermique de médicament selon la revendication 1, comprenant en outre une teneur totale en fentanyl sélectionnée dans le groupe constitué par environ 1,25 milligrammes, environ 2,5 milligrammes, environ 5 milligrammes, environ 7,5 milligrammes, et environ 10 milligrammes.

3. Dispositif d'administration transdermique de médicament selon la revendication 1 ou 2, dans lequel le copolymère comprend au moins deux secondes unités monomères, et dans lequel l'acrylate d'alkyle en $C_4$ à $C_{10}$ et les au moins deux secondes unités monomères constituent ensemble au moins 98 % en poids du copolymère.

**4.** Dispositif d'administration transdermique de médicament selon l'une quelconque des revendications précédentes, dans lequel le copolymère comprend l'acrylate d'alkyle en $C_4$ à $C_{10}$ dans une plage de 60 à 97 % en poids, au moins l'un parmi l'acrylamide ou la N-vinyl-2-pyrrolidone dans une plage de 3 à 10 % en poids, et l'acétate de vinyle dans une plage de 0 à 30 % en poids, dans lequel chaque pourcentage en poids est basé sur le poids total du copolymère.

**5.** Dispositif d'administration transdermique de médicament selon la revendication 1 ou 2, dans lequel le copolymère comprend l'acrylate d'alkyle en $C_4$ à $C_{10}$ dans une plage de 50 à 75 % en poids et l'acétate de vinyle dans une plage de 25 à 50 % en poids, dans lequel chaque pourcentage en poids est basé sur le poids total du copolymère.

**6.** Dispositif d'administration transdermique de médicament selon la revendication 4 ou 5, dans lequel l'acrylate d'alkyle en $C_4$ à $C_{10}$ est l'acrylate d'isooctyle, l'acrylate de 2-éthylhexyle, ou une de leurs combinaisons.

**7.** Dispositif d'administration transdermique de médicament selon l'une quelconque des revendications précédentes, dans lequel la composition adhésive comprend l'activateur de perméation cutanée dans une plage de 13 à 20 % en poids par rapport au poids total de la composition adhésive.

**8.** Dispositif d'administration transdermique de médicament selon l'une quelconque des revendications précédentes, dans lequel l'activateur de perméation cutanée comprend au moins l'un parmi le myristate d'isopropyle, le tétraglycol, le laurate de méthyle, le propylène glycol, le monolaurate de propylène glycol, l'oléate d'éthyle, le 2-octyl-1-dodécanol, le lactate de lauryle, ou l'alcool laurylique.

**9.** Dispositif d'administration transdermique de médicament selon l'une quelconque des revendications précédentes, pour une utilisation dans le traitement d'un état pouvant être traité par le fentanyl chez un mammifère, lequel dispositif d'administration transdermique de médicament est destiné à être employé dans un procédé comprenant :

placer le dispositif d'administration transdermique de médicament sur la peau du mammifère de sorte que la composition adhésive soit en contact avec la peau du mammifère ; et
permettre à la composition adhésive de rester sur la peau pendant un temps suffisant pour établir ou maintenir un niveau sanguin thérapeutiquement efficace de fentanyl chez le mammifère.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4588580 A
- US 20020119187 A, Cantor
- US 20040213832 A, Venkatraman
- US 20060039960 A, Cordes
- US 20040001882 A, Tisa-Bostedt
- US 2003097008, Stefano
- US 4693776 A, Krampe
- US RE24906 E, Ulrich
- US 4732808 A, Krampe
- US 7097853 B, Garbe

**Non-patent literature cited in the description**

- Fentanyl Citrate. AHFS 98 Drug Information. American Society of Health-Systems Pharmacists, 1998, 1677-1683
- **A. POKLIS.** Fentanyl: A Review for Clinical and Analytical Toxicologists. *Clinical Toxicology,* 1995, vol. 33 (5), 439-447